(19) Europäisches Patentamt
European Patent Office
Office européen des brevets

(11) EP 2 556 802 A1

(12) EUROPEAN PATENT APPLICATION
published in accordance with Art. 153(4) EPC

(43) Date of publication:
13.02.2013 Bulletin 2013/07

(51) Int Cl.:
*A61B 17/16* (2006.01)    *A61B 17/56* (2006.01)
*A61B 19/00* (2006.01)    *B23B 39/14* (2006.01)

(21) Application number: 11765481.4

(22) Date of filing: 28.03.2011

(86) International application number:
PCT/JP2011/057562

(87) International publication number:
WO 2011/125581 (13.10.2011 Gazette 2011/41)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 05.04.2010 JP 2010086836

(71) Applicant: NTN Corporation
Osaka-shi, Osaka 550-0003 (JP)

(72) Inventors:
• NISHIO, Yukihiro
Iwata-shi
Shizuoka 438-0037 (JP)
• NAGANO, Yoshitaka
Iwata-shi
Shizuoka 4380078 (JP)

(74) Representative: Hirsch & Associés
58, avenue Marceau
75008 Paris (FR)

(54) REMOTELY OPERATED ACTUATOR

(57) A remote controlled actuator assembly includes an elongated guide section (3), a distal end member (2) fitted to the guide section (3) for alteration in attitude, and an operating tool (1) retained by the distal end member (2). An attitude altering member (31) within the guide section (3) is reciprocally movably inserted by an attitude altering drive source (43) to alter the attitude of the distal end member (2). The attitude altering drive source (43) is controlled by an attitude altering controller (63). An attitude detection unit (9) detects the attitude of the distal end member (2) relative to the guide section (3). A calibrator (65), by use of the attitude information detected by the attitude detection unit (9), corrects or generates a command value which the attitude altering controller (63) outputs to the attitude altering drive source (43).

Fig. 1

EP 2 556 802 A1

**Description**

CROSS REFERENCE TO THE RELATED APPLICATION

This application is based on and claims Convention priority to Japanese patent application No. 2010-086836, filed April 5, 2010, the entire disclosure of which is herein incorporated by reference as a part of this application.

BACKGROUND OF THE INVENTION

(Field of the Invention)

The present invention relates to a remote controlled actuator assembly capable of altering the attitude of a work tool, provided at a tip of an elongated guide section of a curved configuration, by remote control.

(Description of Related Art)

A remote controlled actuator assembly of a type for use in a cutting process in the medical application or in the mechanical application has long been known. This remote controlled actuator assembly is of a structure in which a work tool such as, for example, a tool or a holder element is provided at a tip of an elongated guide section of a straight configuration or a curved configuration and this work tool is controlled by remote control. Hereinafter, in discussing over the problems inherent in the conventional art of the remote controlled actuator assembly, reference will be made to the use of the conventional remote controlled actuator assembly in the medical field in processing a bone.
In the orthopedic field, the artificial joint replacement is well known, in which a joint, of which bone has been abraded by due to bone deterioration, is replaced with an artificial joint. The joint replacement surgery requires a living bone of a patient to be processed to enable an artificial joint to be implanted. In order to enhance the strength of postoperative adhesion between the living bone and the artificial joint, such processing is required to be performed precisely and accurately in conformity to the shape of the artificial joint.
By way of example, during the hip joint replacement surgery, a thigh bone is opened to secure access of an artificial joint into the femoral marrow cavity. In order to secure a strength of contact between the artificial joint and the bone, surfaces of contact of the artificial joint and the bore must be large and so the opening for insertion of the artificial joint is processed to represent an elongated shape extending deep into the bone. In this procedure, a remote controlled actuator assembly is used of a type having such a structure that a tool is rotatably provided in a tip of an elongated guide section so that the tool can be rotated by remote control. The surgical operation for artificial joint replacement generally accompanies skin incision and muscular scission. In other words, the human body must be invaded. In order to minimize the postoperative trace, it is quite often desirable that the elongated guide referred to above is not necessarily straight, but is moderately curved.
To meet this desire, the following technique has hitherto been suggested. For example, the Patent Document 1 listed below discloses the elongated pipe having its intermediate portion curved twice to displace an axial position of the distal end of the pipe relative to the longitudinal axis of the proximal end of the same pipe. To make the axial position of the distal end of the pipe relative to the longitudinal axis of the proximal end of the same pipe is also known from other publications. Also, the Patent Document 2 listed below discloses the elongated pipe rotated by 180°.

[Prior Art Literature]

   [Patent Document 1] United States Patent No. 4,466,429
   [Patent Document 2] United States Patent No. 4,265,231

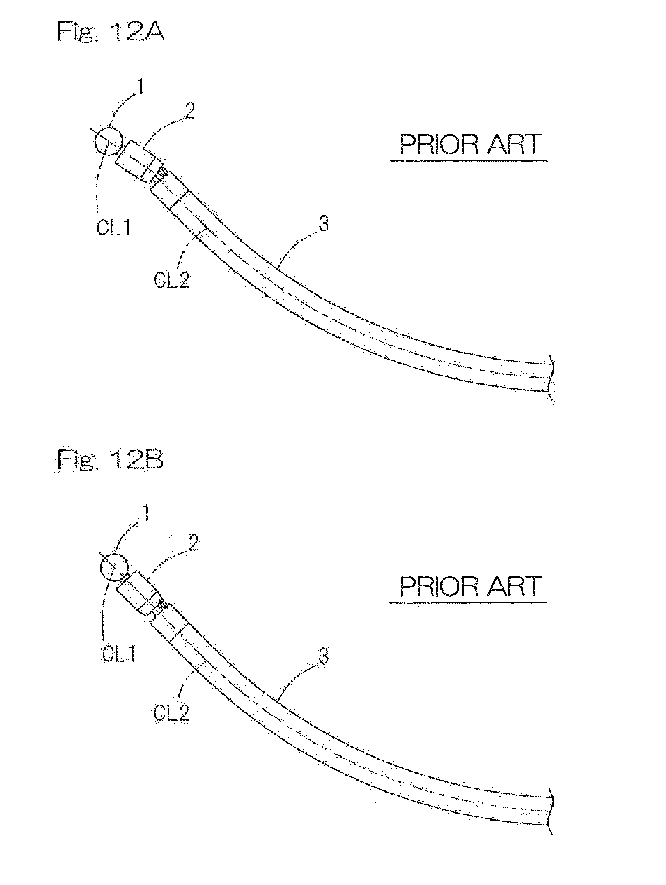

Since the above described remote controlled actuator assembly conventionally employed in the medical field merely controls the rotation of the tool by remote control, accurate positioning of the tool at a site deep into the hole for insertion of the artificial joint and processing of the bone to a complicated shape have been difficult to achieve. In view of this, in order to alleviate the foregoing problems and inconveniences, as shown in Figs. 12A and 12B, the attempt has been made, in which the distal end member 2 for rotatably supporting the tool 1 is provided at the tip of the guide section 3 for alteration in the attitude and the attitude of the distal end member 2 is made alterable by remote control. More specifically, the attempt has been made in which a guide hole (not shown) having its opposite end opening is provided within the guide section 3, an attitude altering member (not shown) of a wire-like shape is inserted within this guide hole for selective advance or retraction and a working force for the attitude alteration is applied to the distal end member 2 through this attitude altering member so that the distal end member 2 can be altered in attitude. If the attitude altering member of the wire-like shape is flexible, the working force necessary to alter the attitude of the distal end member 2 can be transmitted to the distal end member 2 even in the case of the guide section 3 and the guide hole being curved.

In such a remote controlled actuator assembly where an acting force is applied via an attitude altering member to the distal end member 2 for alteration in attitude, a preload applied in advance by the attitude altering member to the distal end member 2 keeps the attitude of the distal end member 2 stable. The preload is just enough to overcome possible external forces that the distal end member 2 may be subjected to. The alteration in attitude of the distal end member 2 is performed by advancing or retracting the attitude altering member according to the desired degree of alteration in attitude with respect to the initial position-attitude of the distal end member 2 and then applying the corresponding acting force to the distal end member.

In general, the neutral attitude of the distal end member 2 is defined as the attitude of the distal end member 2 when the distal end member 2 is aligned straight with the guide section 3 as shown in Fig. 12B - that is, when the center line CL2 of the guide section 3 coincides with the center line CL1 of the distal end member 2. However, it is not unusual that the center line CL2 of the guide section 3 does not completely coincide with the center line CL1 of the distal end member 2 as shown in Fig. 12A, since factors such as processing accuracy and assembly accuracy of components may lead, when the components are assembled, to different remote controlled actuator assemblies having slightly different straightness of the distal end member 2 with respect to the guide section 3. In other words, the advance or retraction position of the attitude altering member corresponding to when the distal end member 2 is actually straight is not necessarily the same as the advance or retraction position of the attitude altering member corresponding to when the distal end member 2 is supposed to be straight according to the design specification.

Thus, to accurately alter the attitude of the distal end member 2 into a target attitude, attitude alteration control of the distal end member 2 must be performed, in which the initial position of the attitude altering member - i.e. the advance or retraction position of the attitude altering member corresponding to when the distal end member 2 is actually straight -is determined for each product and the advance or retraction amount of the attitude altering member is corrected for the difference between that initial position and the advance or retraction position of the attitude altering member corresponding to when the distal end member 2 is supposed to be straight according to the design specification. Alternatively, the relationship between the various attitudes of the distal end member 2 and the corresponding advance or retraction positions of the attitude altering member may be stored in a memory, and the advance or retraction position of the attitude altering member corresponding to the target attitude of the distal end member may be extracted from the stored content in the memory.

## SUMMARY OF THE INVENTION

An object of the invention is to provide a remote controlled actuator assembly which is capable of accurately altering by remote control the attitude of a distal end member mounted to a tip end of an elongated guide section with the distal end member supporting an operating tool, and which can perform appropriate control of alteration in attitude of the distal end member based on the straightness of the distal end member while accommodating possibly different straightness of distal end members among different remote controlled actuator assemblies.

The present invention provides a remote controlled actuator assembly which includes: a guide section of an elongated shape; a distal end member, fitted to a tip end portion of the guide section for alteration in attitude, for retaining an operating tool; an operating tool drive source for driving the operating tool; an attitude altering drive source for altering the attitude of the distal end member; and an attitude altering controller for controlling the attitude altering drive source according to an input value from an attitude alteration operating device. The guide section has its interior accommodating a drive shaft for transmitting a drive force of the operating tool drive source to the operating tool and a guide hole having its opposite ends opening. An attitude altering member is reciprocally movably inserted within the guide hole for undergoing a reciprocating or retracting motion so as to alter the attitude of the distal end member, with the attitude altering member being selectively advanced or retracted by the attitude altering drive source. The remote controlled actuator assembly further includes an attitude detection unit configured to detect, without contacting the distal end member, the attitude of the distal end member relative to the guide section; and a calibrator configured to use attitude information detected by the attitude detection unit to correct or generate a command value which the attitude altering controller outputs, according to an input value provided from the attitude alteration operating device, to the attitude altering drive source.

According to the above described construction, the operating tool provided in the distal end member performs a certain operation. In such case, the attitude altering controller controls the attitude altering drive source according to the input value from an attitude alteration operating device, causing the attitude altering member to advance or retract. By doing so, the attitude altering member works on the distal end member to allow the attitude of the distal end member, fitted to the tip end portion of the guide section for alteration in attitude, to alter. The attitude altering drive source is provided at a position distant from the distal end member and the alteration of the attitude of the distal end member is carried out by remote control. Since the attitude altering member is passed through the guide hole, the attitude altering member can work on the distal end member properly at all time without being displaced in a direction transverse to the longitudinal direction thereof, and the operation to alter the attitude of the distal end member takes place accurately.

Factors such as processing accuracy and assembly accuracy of components may lead, when the components are assembled, to different remote controlled actuator assemblies having slightly different straightness of a distal end member with respect to a guide section. In other words, the advance or retraction position of the attitude altering member corresponding to when the distal end member is straight with respect to the guide section varies among different remote controlled actuator assemblies. To address this, the attitude detection unit detects the attitude of the distal end member relative to the guide section, and the calibrator uses attitude information detected by the attitude detection unit to correct or generate a command value which the attitude altering controller outputs, according to the input value provided from the attitude alteration operating device, to the attitude altering drive source. In this way, appropriate control of alteration in attitude of the distal end member can be performed while accommodating possibly different straightness of distal end members among different remote controlled actuator assemblies. Since the attitude detection unit is configured to detect the attitude of the distal end member without contacting the distal end member, an excellent sanitary condition of the distal end member can be maintained.

In the present invention, the remote controlled actuator assembly preferably includes a memory configured to store, as an initial position of the attitude altering member, an advance or retraction position of the attitude altering member corresponding to when the attitude of the distal end member detected by the attitude detection unit is straight such that the distal end member has the same axis as that of the tip end portion of the guide section, and the calibrator is preferably configured to use the initial position information stored in the memory to correct the command value. Such correction of the command value by using the initial position information stored in the memory makes it possible to perform attitude alteration control with accuracy. Conventionally, the initial attitude of the distal end member is the attitude of the distal end member corresponding to when the attitude of the distal end member is straight such that the distal end member has the same axis as that of the tip end portion of the guide section. Therefore, storage in the memory of, as an initial position of the attitude altering member, the advance or retraction position of the attitude altering member corresponding to when the attitude of the distal end member is straight such that the distal end member is not inclined with respect to the tip end portion of the guide section facilitates correction of the command value by the calibrator.

In the present invention, the remote controlled actuator assembly may include a memory configured to store a relationship between the attitude of the distal end member detected by the attitude detection unit and an advance or retraction position of the attitude altering member, and the calibrator may be configured to generate the command value, based on the stored content in the memory. Such generation of the command value based on the stored content in the memory makes it possible to perform attitude alteration control with accuracy.

In the present invention, the attitude detection unit may include a camera configured to capture an image of the guide section and the distal end member, and an attitude estimator configured to estimate the attitude of the distal end member relative to the guide section, based on the image captured by the camera. Also, the attitude detection unit may include a plurality of cameras configured to capture images of the guide section and the distal end member, from different phases about a center line of the guide section, and an attitude estimator configured to estimate a three-dimensional attitude of the distal end member relative to the guide section, based on a plurality of the images captured by the plurality of the cameras.

For instance, the attitude estimator may be configured to extract, from the image captured by the camera, a sole image of the distal end member and a sole image of the guide section, and to estimate, with the use of techniques to determine a principal axis of inertia, an angle defined between a center line of the sole image of the distal end member and a center line of the sole image of the guide section. Also, the attitude estimator may be configured to repeat a combination of an extract operation and a tilt acquisition operation for an arbitrary number of times, and to estimate a tilt of the image acquired by a final tilt acquisition operation, as a tilt of the distal end member, with the extract operation including determining a tilt of the image, as a whole, captured by the camera, cutting off a segment corresponding to the guide section from the image captured by the camera, with a cutoff line defined by a straight line perpendicular to the determined tilt, and extracting only a segment corresponding to the distal end member, and with the tilt acquisition operation including acquiring a tilt of the extracted image. An attitude estimator with either one of the above discussed configurations allows for accurate estimation of the attitude of the distal end member relative to the guide section, based on the image captured by camera.

When the attitude estimator has any one of the above discussed configurations, a pattern matching technique is preferably used to perform an operation of extracting sole segments from the image captured by the camera. The use of a pattern matching technique facilitates the extract of sole segments from image captured by camera.

In the present invention, the attitude detection unit may include a light projector configured to project a plurality of light strips spaced equal distances apart from each other onto the guide section and the distal end member, camera configured to capture an image of the guide section and the distal end member onto which projection is made by the light projector, and an attitude estimator configured to estimate the attitude of the distal end member relative to the guide section based on distances between respective locations, onto which the plurality of the light strips impinge, on the image captured by the camera. If the distal end member is not at a straight attitude that has the same axis as that of the tip end portion of the guide section with the light projector projecting a plurality of light strips onto the guide section and the distal end member, the distances between respective locations in the guide section onto which the light strips impinges on the image captured by the camera will be different from the distances between respective locations in the distal end member onto which the light strips impinges on the image captured by the camera. Such difference of distances can be used for estimation of the attitude of the distal end member relative to the guide section.

In the present invention, the attitude of the distal end member is preferably detected by the attitude detection unit with a preload being applied to the distal end member by the attitude altering member. The preload applied to the distal end member by the attitude altering member may be, for example, caused by a propulsion force of the attitude altering drive source. The configuration of applying a preload to the distal end member by a propulsion force of the attitude altering drive source already provided in a remote controlled actuator assembly eliminates the need to provide for extra components to apply such a preload.

When the attitude of the distal end member is detected by the attitude detection unit with a preload being applied to the distal end member by the attitude altering member, the attitude detection unit may include a preload application confirmer configured to confirm that the preload is being applied, and the attitude of the distal end member may be detected by the attitude detection unit with the application of preload being confirmed by the preload application confirmer.

The present invention provides a method of using a remote controlled actuator assembly, which method uses a remote controlled actuator assembly according to any one of the aforementioned constructions or configurations. The method includes the steps of: applying a preload to the distal end member by the attitude altering member; and detecting the attitude of the distal end member by means of the attitude detection unit with the preload being applied to the distal end member by the attitude altering member.

BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:

Fig. 1 shows a schematic configuration of a remote controlled actuator assembly according to the first preferred embodiment of the present invention;

Fig. 2A shows a cross sectional view of a distal end member and a guide section of the remote controlled actuator assembly of Fig. 1;

Fig. 2B shows a cross sectional view of Fig. 2A taken along the line IIB-IIB;

Fig. 2C shows a coupling configuration between the distal end member and a drive shaft;

Fig. 2D shows a housing for the distal end member as viewed from a base end of the housing;

Fig. 3 shows a cross sectional view of Fig. 1 taken along the line III-III;

Fig. 4 shows a front elevational view of the distal end member in the remote controlled actuator assembly and cameras to illustrate their positional relationship, along with the image captured by the cameras;

Fig. 5 shows a block diagram of a schematic configuration of a control system of the remote controlled actuator assembly;

Fig. 6A shows an image captured by a camera, illustrating a process of attitude estimation by an attitude estimator in the remote controlled actuator assembly;

Fig. 6B shows another image captured by the camera, illustrating a process of attitude estimation by the attitude estimator in the remote controlled actuator assembly;

Fig. 6C shows yet another image captured by the camera, illustrating a process of attitude estimation by the attitude estimator in the remote controlled actuator assembly;

Fig. 7A shows an image captured by a camera, illustrating a different process of attitude estimation by an attitude estimator;

Fig. 7B shows another image captured by the camera, illustrating a different process of attitude estimation by the attitude estimator;

Fig. 7C shows yet another image captured by the camera, illustrating a different process of attitude estimation by the attitude estimator;

Fig. 7D shows yet another image captured by the camera, illustrating a different process of attitude estimation by the attitude estimator;

Fig. 7E shows yet another image captured by the camera, illustrating a different process of attitude estimation by the attitude estimator;

Fig. 8A shows a side view of a state of an attitude detection unit of a different remote controlled actuator assembly;

Fig. 8B shows a plan view of a state of a distal end member and of a guide section onto which a light is projected by a light projector of the attitude detection unit;

Fig. 9A shows a side view of a different state of an attitude detection unit of a different remote controlled actuator assembly;

Fig. 9B shows a plan view of a state of a distal end member and of a guide section onto which a light is projected by a light projector of the attitude detection unit;

Fig. 10 is a block diagram showing a schematic configuration of part of a control system of a further different remote controlled actuator assembly;

Fig. 11 is a flow chart of control by a table generating controller of the further different remote controlled actuator assembly;

Fig. 12A shows a side view of a state of part of a remote controlled actuator assembly; and

Fig. 12B shows a side view of a state different from Fig. 12A.

DESCRIPTION OF PREFERRED EMBODIMENTS

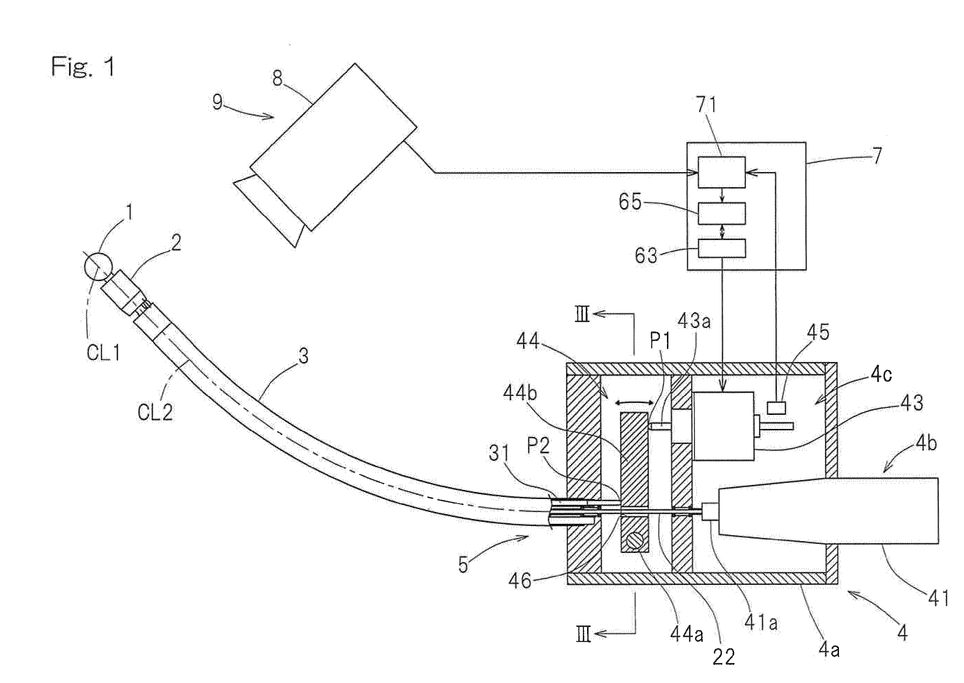

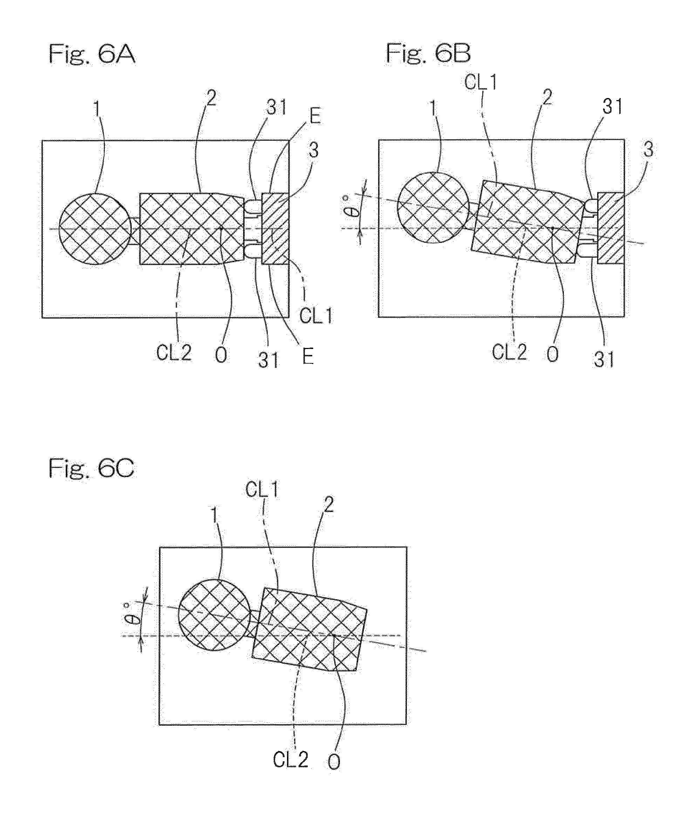

A first preferred embodiment of the present invention will be described in connection with Fig. 1 through Figs. 6A, 6B and 6C. In Fig. 1, a remote controlled actuator assembly includes an actuator body 5, a controller 7 for controlling the actuator body 5, and a camera 8 for detecting the attitude of a distal end member 2 of the actuator body 5. The actuator body 5 and the controller 7 are connected with each other via an electrical cable (not shown). The controller 7 and the camera 8 are connected with each other via an electrical cable (not shown) as well.

The actuator body 5 includes a distal end member 2 for holding an operating tool in the form of a rotary tool 1, a guide section 3 of an elongated, curved shape having a tip end to which the distal end member 2 is fitted for alteration in attitude, and a drive unit housing 4a to which a base end of the guide section 3 is connected. The drive unit housing 4a forms a drive unit 4 together with a built-in operating tool drive mechanism 4b and a similarly built-in attitude altering drive mechanism 4c. In the illustrated embodiment the guide section 3 has a straight line shape at locations closer to the tip end and the base end thereof, and also has an arcuate shape of a substantially constant curvature at intermediate locations. The guide section 3 may have a linear shape along its entire length.

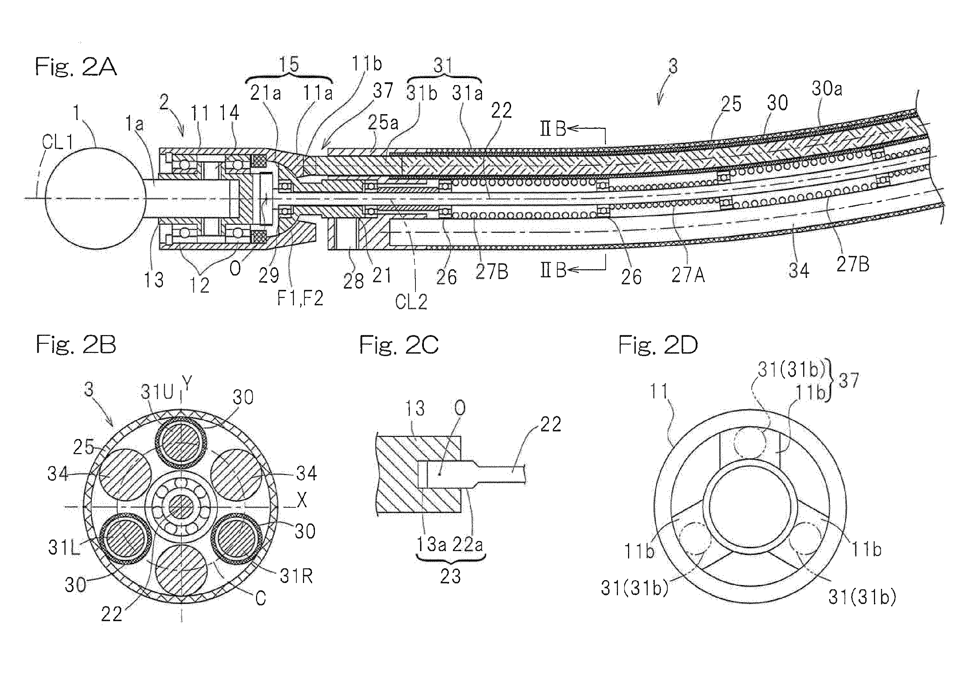

As shown in Fig. 2A, the distal end member 2 includes a generally or substantially cylindrical housing 11 and a spindle 13 rotatably accommodated within such cylindrical housing 11 through a pair of bearings 12. The spindle 13 is of a tubular shape having a distal side opening and has a hollow defined therein, and a tool 1 is drivingly coupled with the spindle 13. Specifically, a shank portion 1a of the tool 1 is inserted into the hollow of the spindle 13 and is then coupled with such spindle 13 by means of a stop pin 14 for rotation together with the spindle 13. The distal end member 2 of the structure described above is coupled with a distal end of the guide section 3 through a distal end member coupling unit 15. The distal end member coupling unit 15 supports the distal end member 2 for displacement in attitude and is comprised of a spherical bearing. More specifically, the distal end member coupling unit 15 includes a guided member 11a in the form of an inner diameter reduced portion at a base end of the housing 11, and a guide member 21a in the form of a collar integral with a constraint member 21 fixed to the tip of the guide section 3. The guided member 11a and the guide member 21a have respective guide faces F1 and F2 that are held in sliding contact with each other, and those guide faces F1 and F2 have respective centers of curvature lying at a point O on the center line or longitudinal axis CL1 of the spindle 13, having their diameters being reduced towards the base end of the spindle 13. Accordingly, not only can the distal end member 2 be immovably constrained relative to the guide section 3, but it can also be supported for displacement in attitude so that the attitude of the distal end member 2 can be altered.

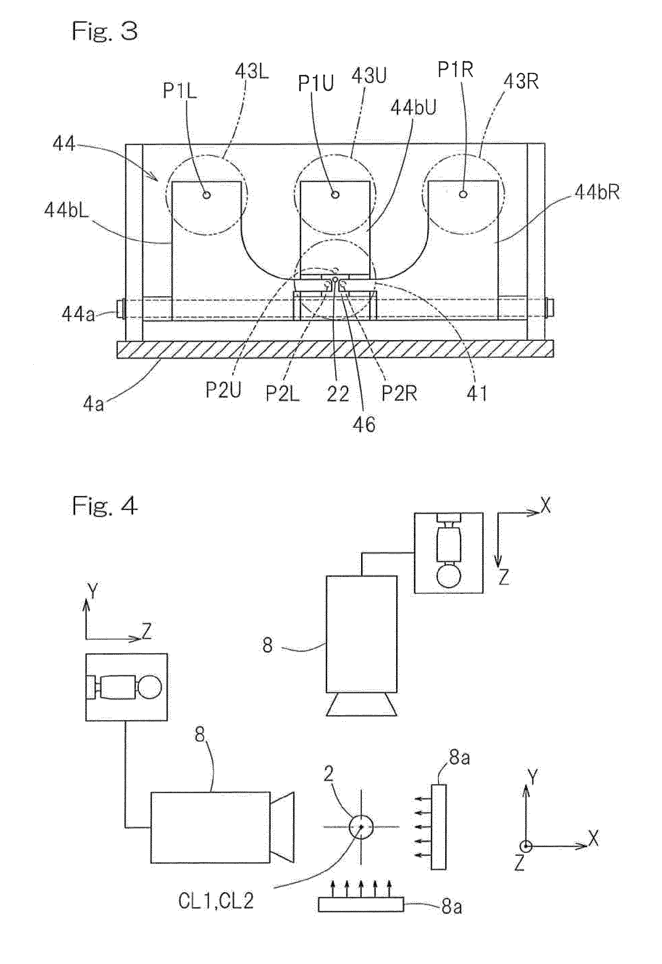

The guide section 3 includes a drive shaft 22 for transmitting a rotational force exerted by a work tool drive source 41 (Figs. 1 and 3) accommodated within the drive unit housing 4a. In the illustrated example, the drive shaft 22 is employed in the form of a wire capable of undergoing deformation to a certain extent. Material for the wire includes, for example, metal, plastic or glass fiber. The wire may be either a single wire or a twisted wire. As best shown in Fig. 2C, the spindle 13 and the drive shaft 22 are coupled together by means of a universal joint 23 for transmitting rotation from the drive shaft 22 to the spindle 13. The universal joint 23 is made up of a groove 13a, defined in a closed base end of the spindle 13, and of a projection 22a defined in a distal end of the drive shaft 22 and engageable in the groove 13a. The center of joint between the groove 13a and the projection 22a is located at the same position as the centers of curvature O of the guide faces F1 and F2. It is to be noted that the drive shaft 22 and the projection 22a may be formed as respective members separate from each other.

As shown in Figs. 2A and 2B, the guide section 3 has an outer shell pipe 25, which forms an outer shell of the guide section 3, and the drive shaft 22 referred to above is positioned at a center of this outer shell pipe 25. The drive shaft 22 so positioned is rotatably supported by a plurality of rolling bearings 26 positioned spaced a distant apart from each other in a direction axially of the guide section 3. Between the neighboring rolling bearings 26, spring elements 27A for generating a preload on the inner rings of the corresponding rolling bearing 26 and spring elements 27B for generating the preload on the outer rings of the corresponding rolling bearings 26 are alternately disposed relative to each other. Those spring elements 27A and 27B may be employed in the form of, for example, compression springs. The constraint member 21 referred to previously is fixed to a pipe end portion 25a of the outer shell pipe 25 by means of a fixing pin 28 and has its distal end inner peripheral portion supporting a distal end of the drive shaft 22 through a rolling bearing 29. It is, however, to be noted that the pipe end portion 25a may be a member separate from the outer shell pipe 25 and may then be connected with the outer shell pipe 25 by means of, for example, welding.

Three guide pipes 30 are arranged between an inner diametric surface of the outer shell pipe 25 and the drive shaft 22 at respective circumferential locations spaced 120° in phase from each other, as shown in Fig. 2B. The guide pipes 30 are in the form of curved shapes and have its opposite ends opening. Within guide holes 30a which are inner diametric holes of these guide pipes 30, attitude altering or operating members 31 (31U, 31L, 31R) are reciprocally movably inserted, respectively. Each of the attitude altering members 31 includes a wire 31a and a column-like pin 31b provided in opposite ends of the wire 31a. For a material for the wire 31a, metal, resin or glass fiber, for example, can be employed. The wire 31a may be either a single wire or a twisted wire. Also, a shape memory alloy may be employed for the wire 31a.

The column-like pin 31b on the side of the distal end member 2 has a tip which is of a spherical shape, and, as shown in Fig. 2D, the spherical tip thereof is held in contact with a bottom surface of a radially extending groove portion 11b formed in the base end face of the housing 11. The groove portion 11b and the attitude altering member 31 altogether form a rotation preventing mechanism 37 for preventing the distal end member 2 from rotating about the center line CL of the distal end member 2 relative to the guide section 3 when the tip portion of the attitude altering member 31, inserted in the groove portion 11b, is engaged with a side face of the groove portion 11b.

Also, as shown in Figs. 2A and 2B, a plurality of reinforcement shafts 34 are arranged, separate from the guide pipe 30, between the inner diametric surface of the outer shell pipe 25 and the drive shaft 22 and on the same pitch circle C as that depicted by the guide pipe 30. Those reinforcement shafts 34 are employed for securing the rigidity of the guide section 3, and have the same diameter and the same curved shape as the guide pipe 30. The guide pipe 30 and the plural reinforcement shafts 34 are spaced an equal distance from each other. The guide pipe 30 and the plural reinforcement shafts 34 are held in contact with the inner diametric surface of the outer shell pipe 25 and an outer diametric surface of each of the rolling bearings 26 so as to support the respective outer diametric surfaces of the rolling bearings 26.

Figs. 1 and 3 illustrate the inside of the drive unit housing 4a. The operating tool drive mechanism 4b includes the tool rotating drive source 41, which is in the form of, for example, an electrically driven motor. The tool rotating drive source 41 is arranged so as to protrude a rear portion thereof from the drive unit housing 4a and have its output shaft 41a coupled with a base end of the drive shaft 22.

The attitude altering drive mechanism 4c includes attitude altering drive sources 43 (43U, 43L and 43R) for selectively advancing or retracting respective attitude altering members 31 (31U, 31L and 31R), best shown in Fig. 2B. Each of the attitude altering dive sources 43 is in the form of, for example, an electrically driven actuator, and the movement of an output rod 43a, which is a direct acting member capable of selectively advancing and retracting, i.e., reciprocally movable in a direction leftwards and rightwards as viewed in Fig. 1, is transmitted to each of the attitude altering members 31 through a force increasing and transmitting mechanism 44. An operating position of the output rod 43a, that is, advancing or retracting position is detected by an attitude detector 45 (45U, 45L and 45R).

The force increasing and transmitting mechanism 44 includes a pivot lever 44b (44bU, 44bL, 44bR) pivotable about a support pin 44a and is so designed and so configured as to allow a force of the output rods 43a to work on a working point P1(P1U, P1L, P1R) of the levers 44b, which are respectively spaced a long distance from the support pin 44a, and as to apply a force to the attitude altering members 31 at a force point P2 (P2U, P2L, P2R), which are spaced a short distance from the support pin 44a, wherefore the outputs of the attitude altering drive sources 43 can be increased and then transmitted to the attitude altering members 31. Since the use of the force increasing and transmitting mechanism 44 is effective to enable a large force to be applied to the attitude altering members 31 even in the linear actuator of a low output capability, the linear actuator can be downsized. The drive shaft 22 referred to above is made to extend through an opening 46 (Fig. 3) defined in the pivot lever 44b.

In Fig. 1, the camera 8 is operable to detect, without contacting the distal end member 2, the attitude of the distal end member 2. The camera 8, together with an attitude estimator 71 which will be discussed later in detail, forms an attitude detection unit 9. The camera 8 is disposed to capture an image containing the distal end member 2 in its entirety and the tip end portion of the guide section 3. Although a single camera 8 is illustrated in Fig. 1, a plurality of cameras 8 (e.g. two cameras) may capture images of the distal end member 2 and the guide section 3, from different phases about a center line CL1 of the distal end member 2 and a center line CL2 of the guide section 3 (e.g. from two-axis directions perpendicular to each other). The attitude estimator 71 will be discussed later.

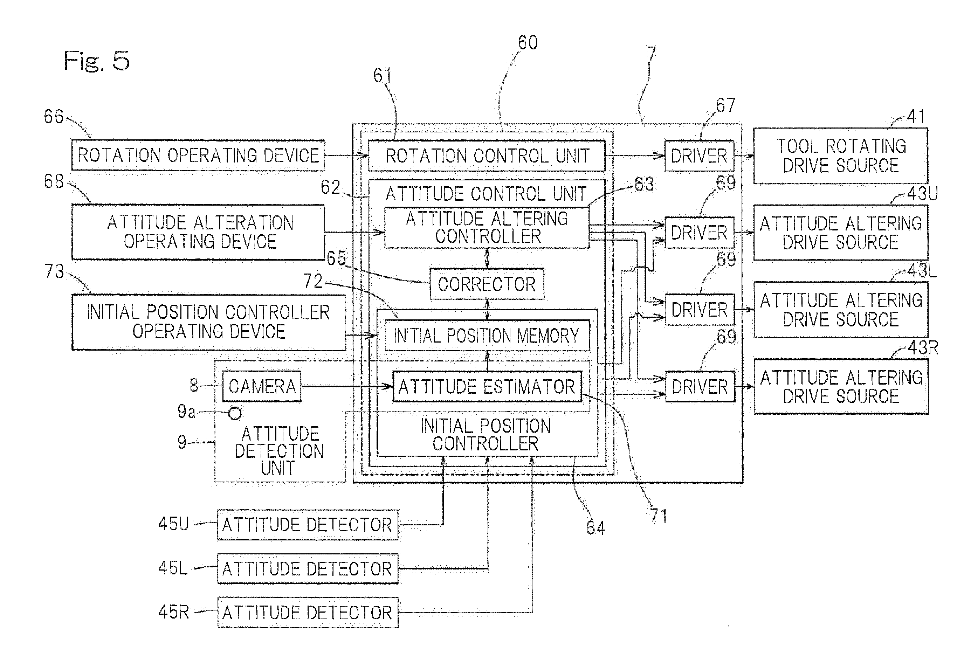

As shown in the block diagram of Fig. 5, the controller 7 is built-in with a computer 60 that performs a variety of controls. The computer 60 includes a rotation control unit 61 configured to control the operating tool drive source 41 and an attitude control unit 62 configured to control the attitude altering drive sources 43(43U, 43L, 43R). The attitude control unit 62 includes an attitude altering controller 63, an initial position controller 64, and a corrector 65. The corrector 65 corresponds to a calibrator which will be discussed later in detail.

The rotation control unit 61 switches on/off the operating tool drive source 41 by generating an output to a driver 67 in response to a rotation command signal from a rotation operating device 66. In this way, the spindle 13 is caused to rotate or to stop rotating. The rotation operating device 66 may be included in the controller 7 or disposed in or on the drive unit housing 4a.

The attitude altering controller 63 of the attitude control unit 62 drives the attitude altering drive sources 43 (43U, 43L, 43R) by generating an output to the drivers 69 in response to an attitude altering command signal from an attitude alteration operating device 68. The attitude alteration operating device 68 is operable to output an attitude alteration command signal that forms the aforementioned input value, for performing an operation of altering the attitude of the distal end member 2 in arbitrary directions and for performing the setting of the degree of that alteration in attitude. The attitude alteration operating device 68 may be included in the controller 7 or disposed in or on the drive unit housing 4a.

By way of example, in the case that the attitude of the distal end member 2 is altered so as to orient the tip end side downwardly as viewed in Fig. 2A, by driving the respective attitude altering drive sources 43, one of the attitude altering members 31U, upper side one as viewed in Fig. 2B, is advanced towards the tip end side while the other two attitude altering members 31L and 31R are retracted. By doing so, the housing 11 for the distal end member 2 is pressed by the upper attitude altering member 31U to allow the distal end member 2 to be altered in attitude along the guide surfaces F1 and F2 with the tip end side consequently oriented downwardly. At this time, those attitude altering drive sources 43 are controlled so that the amount of advance or retraction of each of the attitude altering members 31 may become proper. On the other hand, when each of those attitude altering members 31 is retracted or advanced, the housing 11 for the distal end member 2 is pressed by the attitude altering members 31L and 31R, which are shown on lower left and lower right sides, and, consequently, the distal end member 2 is altered in attitude along the guide surfaces F1 and F2 with the tip end side oriented upwardly.

Also, in the case that the attitude of the distal end member 2 is altered to allow the distal end member 2 to be oriented rightwards as viewed in Fig. 2A, that is, with the distal end member 2 oriented towards a rear side of the sheet of the drawing of Fig. 2A, by driving the attitude altering drive sources 43L, 43R (Fig. 3), the attitude altering member 31L on the left side is advanced towards the tip end side and the attitude altering member 31R on the right side is retracted, while the attitude altering member 31U on the upper side (Fig. 2B) is held still. By doing so, the housing 11 for the distal end member 2 is pressed by the attitude altering member 31 L on the left side to allow the distal end member 2 to be oriented rightwards, that is, to be altered in attitude along the guide surfaces F1 and F2. Conversely, when the attitude altering members 31L and 31R on the left and right sides as viewed in Fig. 2B are advanced and retracted, the housing 11 for the distal end member 2 is pressed by the attitude altering member 31R on the right side, allowing the distal end member 2 to be altered in attitude so that the distal end member 2 can be guided along the guide surfaces F1 and F2 so as to be oriented leftwards.

The initial position controller 64 of the attitude control unit 62, upon the completion of assembly of a remote controlled actuator assembly or upon the replacement of the distal end member 2, carries out initial position control in which an initial position is determined and then stored, which initial position is an advance or retraction position of the attitude altering members 31 corresponding to an initial attitude of the distal end member 2. An initial attitude of the distal end member 2 is defined as the attitude of the distal end member 2 when the distal end member 2 is aligned straight with the guide section 3 - that is, when the center line CL2 of the guide section 3 coincides with the center line CL1 of the distal end member 2. The advance or retraction positions of all of the attitude altering members 31 corresponding to the initial attitude of the distal end member 2 are supposed to be the same, according to the design thereof. However, it is not unusual that the initial positions of all of the attitude altering members 31 are not perfectly the same, since factors such as processing accuracy and assembly accuracy of components may lead, when the components are assembled, to different remote controlled actuator assemblies having slightly different straightness of the distal end member 2 with respect to the guide section 3. Therefore, for each remote controlled actuator assembly, the initial positions of attitude altering members 31 are determined and this initial position information is utilized in the control by the attitude altering controller 63, thereby achieving an attitude alteration control with enhanced accuracy.

The initial position controller 64 includes the attitude estimator 71 and an initial position memory 72. The attitude estimator 71 is configured to estimate the attitude of the distal end member 2 relative to the guide section 3, based on the image of the distal end member 2 and the tip end portion of the guide section 3 captured by the cameras 8. The particular procedures of estimation will be discussed later in detail. The initial position memory 72 is configured to store the initial position of the attitude altering members 31 - i.e. the advance or retraction positions of the attitude altering members 31 corresponding to the initial attitude of the distal end member 2. The initial position controller 64 is connected with an initial position control operating device 73 that is operable to cause initial position control which will be discussed below.

The initial position control operating device 73 is operated to cause initial position control. The attitude altering members 31 are caused to advance starting from their retracted positions, thereby applying a predetermined preload between the distal end member 2 and the attitude altering members 31. This causes the distal end member 2 to be fixed with a certain attitude. The configuration of causing the attitude altering members 31 to advance and apply a preload to the distal end member 2 by a propulsion force of the attitude altering drive sources 43 eliminates the need to provide for extra components to apply such a preload. The attitude detection unit 9 includes a preload application confirmer 9a (Fig. 5) configured to confirm that a preload is being applied to the distal end member 2, and the attitude of the distal end member 2 is detected by the attitude detection unit 9 with the application of preload being confirmed by the preload application confirmer 9a.

As shown in Fig. 4, an image of the distal end member 2 and the tip end portion of the guide section 3 is captured by the cameras 8, and the attitude of the distal end member 2 is estimated by the attitude estimator 71 such as shown in Fig. 5, based on the image captured by these cameras 8. Generally, in a beginning phase, the distal end member 2 is not at an initial attitude. Therefore, the attitude altering members 31 are caused to advance while the attitude of the distal end member 2 is simultaneously being monitored, until the distal end member 2 assumes an initial attitude. The advance or retraction position of the attitude altering members 31 corresponding to the initial attitude of the distal end member 2 is detected, and such a detected value is stored in an initial position memory 72 as an initial position of the attitude altering members 31. The initial position controller 64 automatically carries out these procedures in successive actions. As shown in Fig. 4, parallel light sources 8a are preferably used as light sources, in taking image by the cameras 8. The parallel light source 8a projects a parallel light that is oriented towards the corresponding camera 8 through the distal end member 2 in a parallel fashion to the optical axis of that camera 8.

The corrector 65 uses the initial position information stored in the initial position memory 72 to correct the command value which the attitude altering controller 63 outputs to the attitude altering drive sources 43. The attitude altering controller 63 is configured to output the aforementioned command value to the attitude altering drive sources 43 to cause the attitude altering members 31 to advance or retract. In this way, attitude alteration control can be performed appropriately regardless of different initial positions of the attitude altering members 31 that are different for each product, thereby making it possible to alter the attitude of the distal end member 2 into a target attitude with accuracy.

One process of detecting the attitude of the distal end member 2 by the attitude detection unit 9 will be described in connection with Figs. 6A to 6C. Fig. 6A and Fig. 6B are examples of an image captured by the cameras 8. The attitude of the distal end member 2 with respect to the attitude of the tip end portion of the guide section 3 can be estimated, both when the distal end member 2 is straight with respect to the guide section 3 as shown in Fig. 6A and when the distal end member 2 is bent about the center of curvature O as shown in Fig. 6B. For instance, to estimate the attitude of the distal end member 2 based on the image of Fig. 6B, a sole image of the distal end member 2 is extracted from that image in a manner shown in Fig. 6C. Separation into the image of the distal end member 2 and the image of the guide section 3 is performed by, for example, a pattern matching technique. Then, the following equations (1), (2) for obtaining a principal axis of inertia are used to estimate, based on the extracted image of the distal end member 2, an angle θ defined between a center line CL1 of the distal end member 2 and a center line CL2 of the guide section 3:

$$\theta = \tan^{-1}\left(\frac{2M_{11}}{M_{20} - M_{02}}\right) \qquad (1)$$

$$M_{pq} = \sum_m \sum_n (m - m_G)^p \cdot (n - n_G)^q \cdot f(m,n) \qquad (2)$$

wherein $m_g$ and $n_g$ represent the pixel of the gravity center of the projected image, f (m, n) represents the data (luminance) of respective pixels, m represents a horizontal pixel, and n represents a vertical pixel.

It is noted that p and q represent multiplication factors of $(m-m_G)$ and $(n-n_G)$ in the equation (2), respectively. In the case of $M_{11}$ in the equation (1), the multiplication factors of $(m-m_G)$ and $(n-n_G)$ are one, respectively; thus, $M_{11} = \sum\sum (m-m_G)$ x $(n-n_G)$ x f (m, n). In the case of $M_{20}$, the multiplication factors of $(m-m_G)$ and $(n-n_G)$ are two and zero, respectively; thus, $M_{20} = \sum\sum (m-m_G)$ x $(m-m_G)$ x f (m, n).

In Fig. 6C, $m_g$ and $n_g$, which represent the pixel of the gravity center of an image, correspond to the gravity center of the surface area of the projected image of the distal end member 2 - a point located on the center line CL 1 of the distal end member 2. For example, when the shape of the image of the distal end member 2 is circular or rectangular, $m_g$ and $n_g$ will correspond to the center of that image. However, in Fig. 6C where the distal end member 2 has a varying diameter, $m_g$ and $n_g$ will be offset from the center of the axial length in a left or right direction. Signs m and n representing a pixel will range from 0 to 679 and from 0 to 479, respectively, when the image data has 680 x 480 pixels. Also, for instance, f (m, n) indicates the luminance value of a pixel at a location corresponding to m-1th horizontal position counting from the left end and to n-1th vertical position counting from the upper end.

When the aforementioned procedures are performed for each of the images captured by the two cameras 8 which take images along two-axis directions perpendicular to each other, a three-dimensional attitude of the distal end member 2 can be estimated. Such an optical technique of attitude detection utilizing images of the cameras 8 can detect the attitude of the distal end member 2 without contacting the distal end member 2, thereby maintaining an excellent sanitary condition of the distal end member 2.

The operation of the remote controlled actuator assembly of the structure described hereinabove will now be described. When by operating the rotation operating device 66 the tool rotating drive source 41 as shown in Fig. 5 is driven, the rotational force thereof is transmitted to the spindle 13 through the drive shaft 22 to thereby rotate the tool 1 together with the spindle 13. The tool 1 then being rotated cuts a bone or the like. During processing, the attitude altering drive source 43 is driven by operating the attitude alteration operating device 68 in accordance with a shape of a to-be-processed portion or a progress status of the processing so as to alter the attitude of the distal end member 2 in an arbitrary direction through the attitude altering members 31.

The attitude altering drive source 43 is disposed apart from the distal end member 2, and therefore, the attitude of the distal end member 2 is altered by remote control. Since the attitude altering member 31 is inserted through the guide hole 30a of the guide pipe 30, the attitude altering member 31 can properly act on the distal end member 2 at all times without being accompanied by displacement in position in a direction perpendicular to the lengthwise direction thereof and the attitude altering operation of the distal end member 2 can therefore be performed accurately. Also, since the center of the junction between the spindle 13 and the drive shaft 22 lies at the same position as the respective centers of curvature O of the guide faces F1 and F2, no force tending to press and pull will not act on the drive shaft 22 as a result of the alteration of the attitude of the distal end member 2 and the distal end member 2 can be smoothly altered in attitude.

In addition, since the use has been made of the rotation preventing mechanism 37 for preventing the distal end member 2 from rotating about the center line CL1 of the distal end member 2 relative to the guide section 3, even in the event that the distal end member 2 for holding the tool 1 becomes uncontrollable because of, for example, a failure of one or both of the attitude altering drive mechanism 4c and a control device therefor, the risk can be avoided that the distal end member 2 will rotate about the center line CL1 to impair the surroundings of the site to be processed and/or the distal end member 2 itself may be damaged.

The remote controlled actuator assembly of the foregoing construction is utilized in grinding the femoral marrow cavity during, for example, the artificial joint replacement surgery and during the surgery, it is used with the distal end member 2 in its entirety or a part thereof inserted into the body of a patient. Because of this, with such distal end member 2 as described above that can be altered in attitude by remote control, the bone can be processed in a condition with the tool 1 maintained in a proper attitude at all times and the opening for insertion of the artificial joint can be finished accurately and precisely.

There is the necessity that the drive shaft 22 and the attitude altering member 31 are provided within the guide section 3 of an elongated shape in a protected fashion. Hence, the drive shaft 22 is provided in the center portion of the outer shell pipe 25 and the guide pipe 30, in which the attitude altering member 31 is accommodated, and the reinforcement shafts 34 are arranged between the outer shell pipe 25 and the drive shaft 22 so as to be juxtaposed in the circumferential direction. Accordingly, it is possible to protect the drive shaft 22 and the attitude altering member 31 and, at the same time, the interior can be made hollow to thereby reduce the weight without sacrificing the rigidity. Also, the balance as a whole is good.

Since the outer diametric surfaces of the rolling bearings 26 supporting the drive shaft 22 are supported by the guide pipes 30 and the reinforcement shafts 34, the outer diametric surfaces of the rolling bearings 26 can be supported with no need to use any extra member. Also, since the preload is applied to the rolling bearings 26 by means of the spring elements 27A and 27B, the drive shaft 22 comprised of the wire can be rotated at a high speed. Because of that, the processing can be accomplished with the spindle 13 rotated at a high speed and a good finish of the processing can also be obtained and the cutting resistance acting on the tool 1 can be reduced. Since the spring elements 27A and 27B are disposed between the neighboring rolling bearings 26, the spring elements 27A and 27B can be provided with no need to increase the diameter of the guide section 3.

An initial position attitude of the distal end member 2, which can be defined as an attitude of the distal end member 2 that is straight and has no tilt with respect to the tip end portion of the guide section 3, can be estimated based on the imaged tip end portion such as shown in Fig. 6A. First, the image of the tip end portion of the guide section 3 is extracted, and then, an edge E corresponding to a side edge of the tip end portion of the guide section 3 is detected with the side edge including substantially straight segments. The edge E is either an upper edge of the image of the guide section 3 or a lower edge of the image of the guide section 3. The edge E is approximated by a straight line, and then, the attitude is estimated from the straight line information. In other words, the straight segments information of the edge E is used to estimate the attitude of the tip end portion of the guide section 3, and then, with reference to such an estimated attitude, the attitude of the distal end member 2 is estimated. An initial position attitude of the distal end member 2 can be defined as an attitude of the distal end member 2 having the same axis as that of the tip end portion of the guide section 3. Hence, it is necessary to estimate the attitude of the guide section 3 from the straight segments information of the edge E of the guide section 3 and to determine, with reference to such an estimated attitude, the relative angle of the distal end member 2, thereby estimating the angle of the distal end member 2.

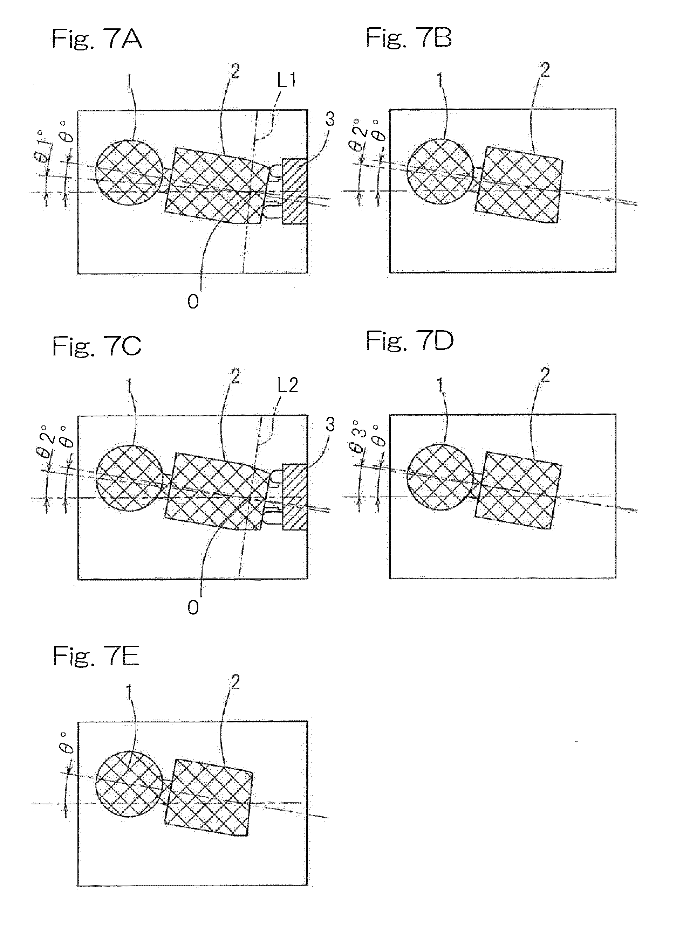

Another process of detecting the attitude of the distal end member 2 by the attitude detection unit 9 will be described in connection with Figs. 7A to 7E. First, a tilt angle θ1 of the total image containing the distal end member 2 and the tip end portion of the guide section 3 is determined. The determined tilt angle θ1 includes deviation from an actual tilt angle θ of the distal end member 2. A segment corresponding to the guide section 3 is cut off from the total image with a cutoff line defined by a straight line L1 perpendicular to the tilt angle θ1 and passing through the center of curvature O, and then, a sole image of the distal end member 2 with a tool 1 is extracted such as shown in Fig. 7B. The tilt angle θ2 of that extracted image is determined. Furthermore, in a manner such as shown in Fig. 7C, a segment corresponding to the guide section 3 is again cut off from the original total image with a cutoff line defined by a straight line L2 perpendicular to the tilt angle θ2 and passing through the center of curvature O, and then, a sole image of the distal end member 2 is extracted such as shown in Fig. 7D. The tilt angle θ3 of that extracted image is determined.

In this way, a combination of an extract operation and a tilt acquisition operation is repeatedly performed for an arbitrarily defined number of times, with the extract operation extracting a sole image of the distal end member 2 from the total image and the tilt acquisition operation acquiring a tilt of the extracted image, and a tilt of the image acquired by a final tilt acquisition operation is estimated as a tilt of the distal end member 2. A repeatedly performing the combination of an extract operation and a tilt acquisition operation will bring the resulting tilt angle gradually closer to the actual tile angle θ and will eventually cause the resulting tilt angle to substantially coincide with the actual tile angle θ as shown in Fig. 7E.

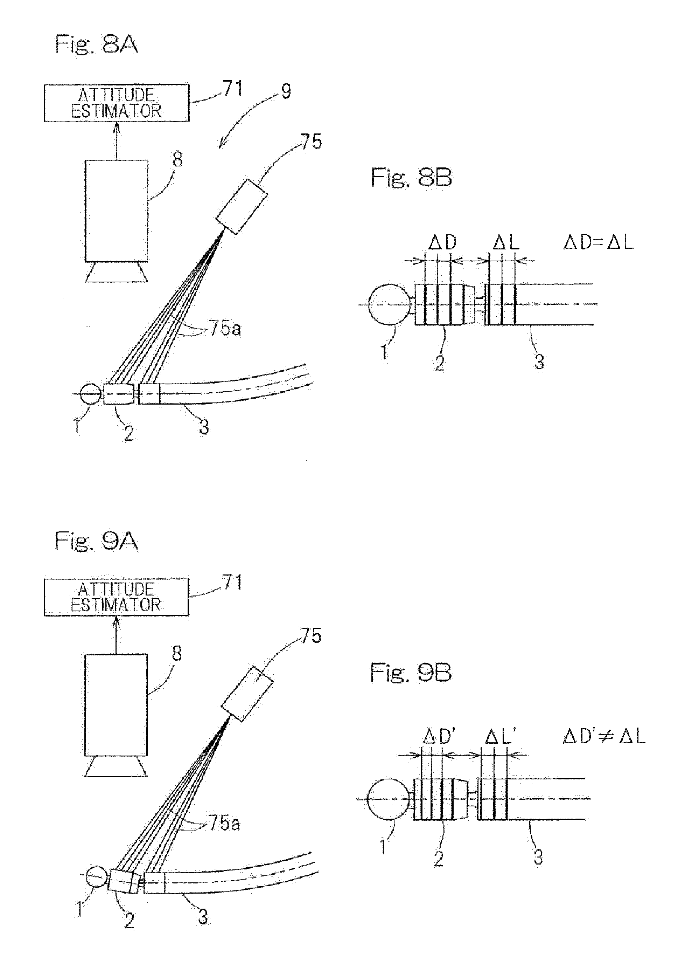

Yet another process of detecting the attitude of the distal end member 2 by the attitude detection unit 9 will be described in connection with Figs. 8A to 9B. The attitude detection unit 9 includes, in addition to the cameras 8 and the attitude estimator 71, a light projector 75 configured to project a plurality of light strips 75a spaced equal distances apart from each other, onto the straight tip end portion of the guide section 3 and the distal end member 2. When the light projector 75 projects a plurality of light strips 75a onto the tip end portion of the guide section 3 and the distal end member 2, with the distal end member 2 being at an attitude straight with respect to the tip end portion of the guide section 3 as shown in Fig. 8A, the distances ΔL between respective locations in the guide section 3, onto which the light strips impinges, on the image captured by the cameras 8 will be the same as the distances ΔD between respective locations in the distal end member 2, onto which the light strips impinges, on the image captured by the cameras 8, as shown in Fig. 8B. On the other hand, when the distal end member 2 is not at an attitude straight with respect to the tip end portion of the guide section 3 as shown in Fig. 9A, the distances ΔL' between respective locations in the guide section 3, onto which the light strips impinges, on the image captured by the cameras 8 will be different from the distances ΔD' between respective locations in the distal end member 2, onto which the light strips impinges, on the image captured by the cameras 8, as shown in Fig. 9B. From such difference of distances the attitude of the distal end member 2 relative to the guide section 3 can be estimated by the attitude estimator 71.

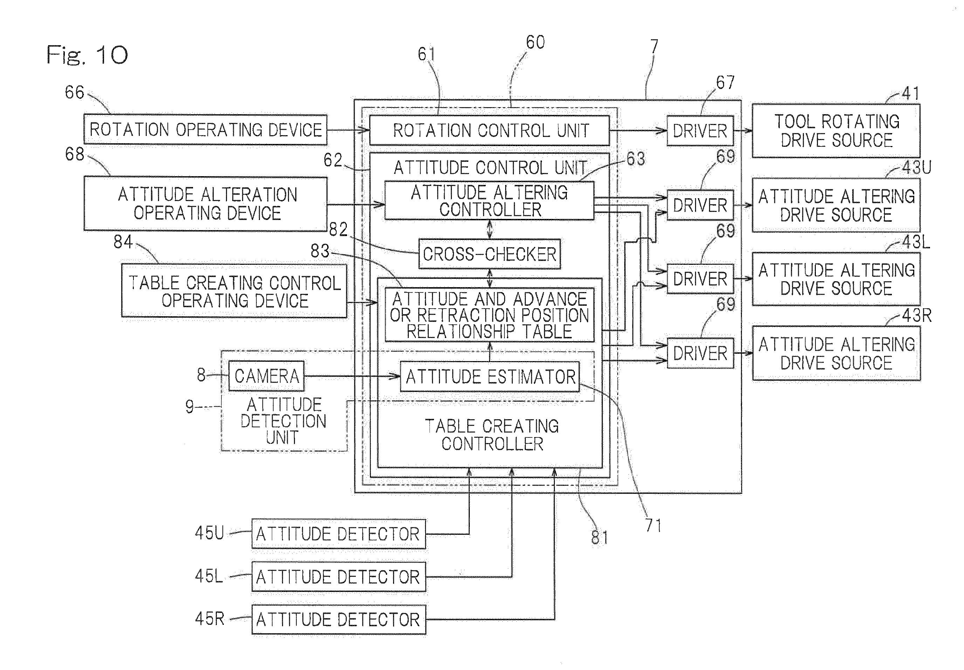

Also, the attitude control unit 62 may have a configuration such as shown in Fig. 10. The attitude control unit 62 includes an attitude altering controller 63, a table generating controller 81, and a cross-checker 82. The cross-checker 82 is a calibrator, just as the aforementioned corrector 65 is. The table generating controller 81 includes the attitude estimator 71 and an advance or retraction position relationship table 83. The advance or retraction position relationship table 83 is a memory configured to store a relationship between the attitude of the distal end member 2 detected by the attitude detection unit 9 and an advance or retraction position of the attitude altering members 31. The attitude estimator 71 has any one of the configurations as discussed above. The table generating controller 81 is connected with a table generation control operating device 84 operable to actuate table generating control which will be discussed below.

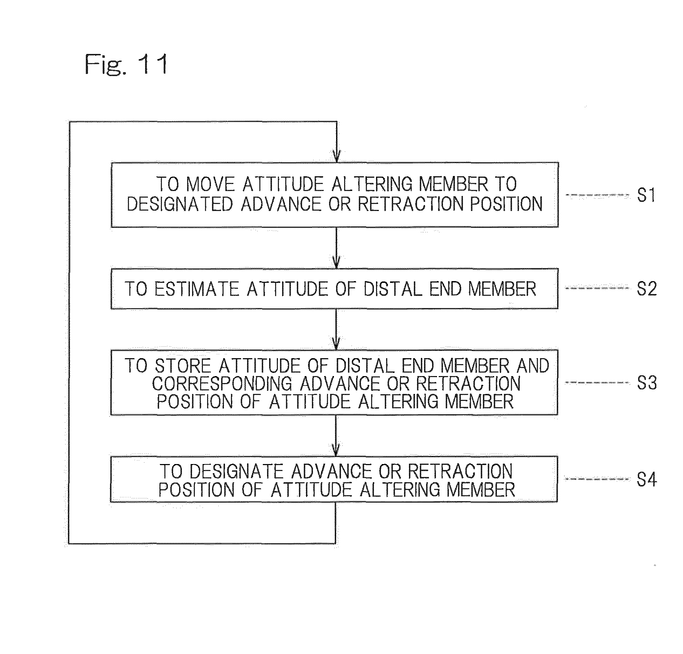

The table generation control operating device 84 is operated to cause the table generating controller 81 to perform table generating control such as illustrated in a flow chart of Fig. 11. In particular, the attitude altering members 31 are moved to a designated advance or retraction position (S1). The attitude estimator 71 estimates the attitude of the distal end member 2 based on the image captured by the cameras 8 (S2). The estimated attitude of the distal end member 2 as well as the corresponding advance or retraction position of the attitude altering members 31 are stored in the advance or retraction position relationship table 83 (S3). Another advance or retraction position of the attitude altering member 31 is designated (S4). Then, the moving of the attitude altering members 31 (S1), the estimation of the attitude of the distal end member 2 (S2), the storage of the estimated attitude of the distal end member 2 as well as the corresponding advance or retraction position of the attitude altering members 31 (S3), and the designation of the advance or retraction position of the attitude altering members 31 (S4) are repeatedly carried out, and data indicative of the relationship between the advance or retraction position of the attitude altering members 31 and the attitude of the distal end member 2 is generated in the advance or retraction position relationship table 83.

The cross-checker 82 is configured to, upon receipt of a request from the attitude altering controller 63, cross-check the data stored in the advance or retraction position relationship table 83, extract the advance or retraction position of the attitude altering members 31 that corresponds to the attitude of the distal end member 2 designated by the attitude alteration operating device 68, and generate a command value for output to the attitude altering drive sources 43. The attitude altering controller 63 is configured to output the aforementioned command value to the attitude altering drive sources 43 to cause the attitude altering members 31 to advance or retract. In this way, attitude alteration control can be performed appropriately regardless of different straightness of the attitude altering members 31 that is different for each product, thereby making it possible to alter the attitude of the distal end member 2 into a target attitude with accuracy.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention. By way of example, although in any one of the embodiments of the present invention hereinbefore fully described, the work tool has been shown and described as represented by a tool 1, the work tool may be any other work tool such as, for example, a prehension orthosis.

Also, the present invention may not be necessarily limited to the remote controlled actuator assembly for medical use, but can be equally applied to any other remote controlled actuator assembly that is used in any other field such as, for example, a mechanical processing field.

Accordingly, such changes and modifications are, unless they depart from the scope of the present invention as delivered from the claims annexed hereto, to be construed as included therein.

[Reference Numerals]

1 ···· Tool (Operating Tool)
2 ···· Distal end member
3 ···· Guide section
8 ···· Camera
9 ···· Attitude detection unit
9a ···· Preload application confirmer
22 ···· Drive shaft
30 ···· Guide pipe
30a ···· Guide hole
31 ···· Attitude altering member
41 ···· Operating tool drive source
43 ···· Attitude altering drive source
62 ···· Attitude control unit
63 ···· Attitude altering controller
65 ···· Corrector (Calibrator)
68 ···· Attitude alteration operating device
71 ···· Attitude estimator
72 ···· Initial position memory
75 ···· Light projector
75a ··· Light strips
82 ···· Cross-checker (Calibrator)
83 ···· Advance or retraction position relationship table (Memory)

**Claims**

1. A remote controlled actuator assembly which comprises:

   a guide section of an elongated shape;
   a distal end member for retaining an operating tool, the distal end member being fitted to a tip end portion of the guide section for alteration in attitude;
   an operating tool drive source for driving the operating tool;
   an attitude altering drive source for altering the attitude of the distal end member; and

an attitude altering controller for controlling the attitude altering drive source according to an input value from an attitude alteration operating device;

in which the guide section has its interior accommodating a drive shaft for transmitting a drive force of the operating tool drive source to the operating tool and a guide hole having its opposite ends opening, and

in which an attitude altering member is reciprocally movably inserted within the guide hole for undergoing a reciprocating or retracting motion so as to alter the attitude of the distal end member, the attitude altering member being selectively advanced or retracted by the attitude altering drive source;

further comprising:

an attitude detection unit configured to detect, without contacting the distal end member, the attitude of the distal end member relative to the guide section; and

a calibrator configured to use attitude information detected by the attitude detection unit to correct or generate a command value which the attitude altering controller outputs, according to the input value provided from the attitude alteration operating device, to the attitude altering drive source.

2. The remote controlled actuator assembly as claimed in claim 1, further comprising a memory configured to store, as an initial position of the attitude altering member, an advance or retraction position of the attitude altering member corresponding to when the attitude of the distal end member detected by the attitude detection unit is straight such that the distal end member has the same axis as that of the tip end portion of the guide section, wherein the calibrator is configured to use the initial position information stored in the memory to correct the command value.

3. The remote controlled actuator assembly as claimed in claim 1, further comprising a memory configured to store a relationship between the attitude of the distal end member detected by the attitude detection unit and an advance or retraction position of the attitude altering member, wherein the calibrator is configured to generate the command value, based on the stored content in the memory.

4. The remote controlled actuator assembly as claimed in claim 1, in which the attitude detection unit includes a camera configured to capture an image of the guide section and the distal end member, and an attitude estimator configured to estimate the attitude of the distal end member relative to the guide section, based on the image captured by the camera.

5. The remote controlled actuator assembly as claimed in claim 1, in which the attitude detection unit includes a plurality of cameras configured to capture images of the guide section and the distal end member, from different phases about a center line of the guide section, and an attitude estimator configured to estimate a three-dimensional attitude of the distal end member relative to the guide section, based on a plurality of the images captured by the plurality of the cameras.

6. The remote controlled actuator assembly as claimed in claim 4, in which the attitude estimator is configured to extract, from the image captured by the camera, a sole image of the distal end member and a sole image of the guide section, and to estimate, with the use of techniques to determine a principal axis of inertia, an angle defined between a center line of the sole image of the distal end member and a center line of the sole image of the guide section.

7. The remote controlled actuator assembly as claimed in claim 4, in which the attitude estimator is configured to repeat a combination of an extract operation and a tilt acquisition operation for an arbitrary number of times, and to estimate a tilt of the image acquired by a final tilt acquisition operation, as a tilt of the distal end member, the extract operation including determining a tilt of the image, as a whole, captured by the camera, cutting off a segment corresponding to the guide section from the image captured by the camera, with a cutoff line defined by a straight line perpendicular to the determined tilt, and extracting only a segment corresponding to the distal end member, the tilt acquisition operation including acquiring a tilt of the extracted image.

8. The remote controlled actuator assembly as claimed in claim 6, in which a pattern matching technique is used to perform an operation of extracting sole segments from the image captured by the camera.

9. The remote controlled actuator assembly as claimed in claim 1, in which the attitude detection unit includes a light projector configured to project a plurality of light strips spaced equal distances apart from each other onto the guide section and the distal end member, a camera configured to capture an image of the guide section and the distal end member onto which projection is made by the light projector, and an attitude estimator configured to estimate the attitude of the distal end member relative to the guide section based on distances between respective locations, onto which the plurality of the light strips impinge, on the image captured by the camera.

10. The remote controlled actuator assembly as claimed in claim 1, in which the attitude of the distal end member is detected by the attitude detection unit, with a preload being applied to the distal end member by the attitude altering member.

11. The remote controlled actuator assembly as claimed in claim 10, in which the preload applied to the distal end member by the attitude altering member is caused by a propulsion force of the attitude altering drive source.

12. The remote controlled actuator assembly as claimed in claim 10, in which the attitude detection unit includes a preload application confirmer configured to confirm that the preload is being applied, and the attitude of the distal end member is detected by the attitude detection unit with the application of preload being confirmed by the preload application confirmer.

13. A method of using the remote controlled actuator assembly as claimed in claim 1, the method comprising the steps of:

applying a preload to the distal end member by the attitude altering member; and
detecting the attitude of the distal end member by means of the attitude detection unit with the preload being applied to the distal end member by the attitude altering member.

Fig. 1

Fig. 2A

Fig. 2B

Fig. 2C

Fig. 2D

EP 2 556 802 A1

Fig. 3

Fig. 4

Fig. 5

EP 2 556 802 A1

EP 2 556 802 A1

Fig. 6A

Fig. 6B

Fig. 6C

19

Fig. 7A

Fig. 7B

Fig. 7C

Fig. 7D

Fig. 7E

Fig. 8A

ATTITUDE ESTIMATOR — 71

9

75

8

75a

1   2   3

Fig. 8B

$\Delta D$   $\Delta L$   $\Delta D = \Delta L$

1   2   3

Fig. 9A

ATTITUDE ESTIMATOR — 71

75

8

75a

1   2   3

Fig. 9B

$\Delta D'$   $\Delta L'$   $\Delta D' \neq \Delta L$

1   2   3

Fig. 10

66 ROTATION OPERATING DEVICE

68 ATTITUDE ALTERATION OPERATING DEVICE

84 TABLE CREATING CONTROL OPERATING DEVICE

8 CAMERA

9 ATTITUDE DETECTION UNIT

61 ROTATION CONTROL UNIT

62 ATTITUDE CONTROL UNIT

63 ATTITUDE ALTERING CONTROLLER

82 CROSS-CHECKER

83 ATTITUDE AND ADVANCE OR RETRACTION POSITION RELATIONSHIP TABLE

ATTITUDE ESTIMATOR

TABLE CREATING CONTROLLER

67 DRIVER

69 DRIVER

69 DRIVER

69 DRIVER

41 TOOL ROTATING DRIVE SOURCE

43U ATTITUDE ALTERING DRIVE SOURCE

43L ATTITUDE ALTERING DRIVE SOURCE

43R ATTITUDE ALTERING DRIVE SOURCE

45U ATTITUDE DETECTOR

45L ATTITUDE DETECTOR

45R ATTITUDE DETECTOR

EP 2 556 802 A1

Fig. 11

```
    ┌──────────────────────────────────────────────┐
    │   TO MOVE ATTITUDE ALTERING MEMBER TO         │ ---------- S1
    │ DESIGNATED ADVANCE OR RETRACTION POSITION     │
    └──────────────────────────────────────────────┘
                         │
                         ▼
    ┌──────────────────────────────────────────────┐
    │   TO ESTIMATE ATTITUDE OF DISTAL END MEMBER   │ ---------- S2
    └──────────────────────────────────────────────┘
                         │
                         ▼
    ┌──────────────────────────────────────────────┐
    │ TO STORE ATTITUDE OF DISTAL END MEMBER AND    │
    │   CORRESPONDING ADVANCE OR RETRACTION         │ ---------- S3
    │  POSITION OF ATTITUDE ALTERING MEMBER         │
    └──────────────────────────────────────────────┘
                         │
                         ▼
    ┌──────────────────────────────────────────────┐
    │   TO DESIGNATE ADVANCE OR RETRACTION          │ ---------- S4
    │  POSITION OF ATTITUDE ALTERING MEMBER         │
    └──────────────────────────────────────────────┘
```

Fig. 12A

PRIOR ART

Fig. 12B

PRIOR ART

<table>
<tr><td colspan="2">**INTERNATIONAL SEARCH REPORT**</td><td>International application No.<br>PCT/JP2011/057562</td></tr>
</table>

| | |
|---|---|
| **A. CLASSIFICATION OF SUBJECT MATTER**<br>*A61B17/16*(2006.01)i, *A61B17/56*(2006.01)i, *A61B19/00*(2006.01)i, *B23B39/14* (2006.01)i ||
| According to International Patent Classification (IPC) or to both national classification and IPC ||

**B. FIELDS SEARCHED**

Minimum documentation searched (classification system followed by classification symbols)
A61B17/16, A61B17/56, A61B19/00, B23B39/14, B25J1/00-21/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2010-63656 A  (NTN Corp.),<br>25 March 2010 (25.03.2010),<br>claims 1 to 4; paragraphs [0034] to [0038];<br>fig. 1<br>& WO 2010/029741 A1 | 1-5,10-12 |
| Y | JP 2004-50356 A  (Kawasaki Heavy Industries, Ltd.),<br>19 February 2004 (19.02.2004),<br>paragraphs [0019], [0024] to [0027], [0043] to [0047]; fig. 1 to 2, 5<br>(Family: none) | 1-5,10-12 |

☐ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>13 April, 2011 (13.04.11) | Date of mailing of the international search report<br>26 April, 2011 (26.04.11) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2011/057562 |

**Box No. II**     **Observations where certain claims were found unsearchable (Continuation of item 2 of first sheet)**

This international search report has not been established in respect of certain claims under Article 17(2)(a) for the following reasons:

1. ☒  Claims Nos.:  13
      because they relate to subject matter not required to be searched by this Authority, namely:
      Claim 13 pertains to "a method for treatment of the human body by therapy" and thus relates to a subject matter which this International Searching Authority is not required, under the provisions of PCT Rule 39.1(iv), to search.

2. ☐  Claims Nos.:
      because they relate to parts of the international application that do not comply with the prescribed requirements to such an extent that no meaningful international search can be carried out, specifically:

3. ☐  Claims Nos.:
      because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:

1. ☐  As all required additional search fees were timely paid by the applicant, this international search report covers all searchable claims.

2. ☐  As all searchable claims could be searched without effort justifying additional fees, this Authority did not invite payment of additional fees.

3. ☐  As only some of the required additional search fees were timely paid by the applicant, this international search report covers only those claims for which fees were paid, specifically claims Nos.:

4. ☐  No required additional search fees were timely paid by the applicant.   Consequently, this international search report is restricted to the invention first mentioned in the claims; it is covered by claims Nos.:

**Remark on Protest**     ☐  The additional search fees were accompanied by the applicant's protest and, where applicable, the payment of a protest fee.

                          ☐  The additional search fees were accompanied by the applicant's protest but the applicable protest fee was not paid within the time limit specified in the invitation.

                          ☐  No protest accompanied the payment of additional search fees.

Form PCT/ISA/210 (continuation of first sheet (2)) (July 2009)

**EP 2 556 802 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010086836 A
- US 4466429 A
- US 4265231 A